(19)

Europäisches Patentamt
European Patent Office
Office européen des brevets

(11) **EP 3 708 360 A1**

(12) **EUROPEAN PATENT APPLICATION**

(43) Date of publication:
16.09.2020 Bulletin 2020/38

(51) Int Cl.:
**B32B 9/02** (2006.01)      **G01N 27/327** (2006.01)
**G01N 33/543** (2006.01)

(21) Application number: **19161929.5**

(22) Date of filing: **11.03.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Instituto Superior de Engenharia do Porto**
**4249-015 Porto (PT)**

(72) Inventors:
• **Sales, Maria**
  **4470-719 Vila Nova da Telha (PT)**
• **Moreira, Ana**
  **4250-084 Porto (PT)**

(74) Representative: **Oliveira Lourenço, Nuno Miguel**
**Lusoworld II**
**A 25, Rua Pé de Mouro**
**2710-144 Sintra (PT)**

(54) **PRINTED ELECTRODES ON CORK SUBSTRATES AND PROCESS FOR OBTAINING THEM**

(57)    The present invention relates to a cork substrate for printing electrodes, to a cork support comprising electrodes for producing sensing surfaces intended for the electrical transduction of biomolecules, to a biosensor comprising a cork support and to a process for obtaining said substrates, supports and biosensors.

The electrodes are designed on the cork substrates by screen printing techniques. The resulting cork support is suitable for the development of biosensors for any target compound, displaying better electrical features than other supports, while being a low cost and an environmentally friendly approach.

Therefore, the present invention is in the area of devices and materials for analysing biomolecules by the use of electrochemical means.

Figure 2

EP 3 708 360 A1

## Description

### Technical domain of the invention

The present invention relates to a cork substrate for printing electrodes, to a cork support comprising electrodes for producing sensing surfaces intended for the electrical transduction of biomolecules, to a biosensor comprising a cork support and to a process for obtaining said substrates, supports and biosensors.

The electrodes are designed on the cork substrates by screen printing techniques. The resulting cork support is suitable for the development of biosensors for any target compound, displaying better electrical features than other supports, while being a low cost and an environmentally friendly approach.

Therefore, the present invention is in the area of devices and materials for analysing biomolecules by the use of electrochemical means.

### Background of the invention

Widening point-of-care (POC) analysis is today a worldwide target in health. A medical decision is often supported by the levels of several biochemicals in the blood, saliva or urine, and it would benefit from shifting the conventional laboratory tests to the doctor's office. POC testing can also be more accurate by avoiding analyte changes during sample transport/storage, caused by delayed release of analytes, by continued metabolism, and by protein/peptide degradation in whole blood.

POC testing have also been widely applied in environmental contaminants, namely to detect antibiotics, in natural water system, since a considerable amount of antibiotics are liberated into wastewaters via biological fluids from humans and animals, and contamination from animal feeding systems.

The devices involved in POC testing should be portable, small, easy to use and carry, and inexpensive. For this, common POC testing devices, such as those of glucose readings, make use of screen-printed electrodes (SPEs) technology. The number of papers published in literature on SPEs and their citations has observed an exponential growth, since 1992 (Ahmed et al. 2016).

Moreover, a recent study made by IDTechEx forecasts the market for fully printed sensors will be over $8 billion by 2025 (Dr. Guillaume Chansin, IDTechEx Research Report "OLED Display Forecasts 2015-2025: The Rise of Plastic and Flexible Displays". 2015).

Screen-printed technology consists of layer-by-layer depositions of ink upon a solid substrate defining the geometry of the intended sensor. In detail, it makes use of a suitable stencil (mask) to limit the printing areas and uses a mesh to transfer the ink onto the free substrate surface. A blade is then moved across to fill the open apertures with ink and when the stencil is pulled out the ink remains on the substrate, preserving its printed shape. Subsequent stencil shapes are also possible, allowing overlapping several ink layers with different formats.

Overall, this approach has yielded the automated fabrication of commercial SPE devices, containing mostly a three-electrode system (working, auxiliary and reference electrodes), printed on a solid substrate of planar format (Ahmed et al. 2016)[1]. This three-electrode system is ideal for POC analyses, because it allows a reduced sample volume.

A critical point in assembling SPEs is the ink, usually containing carbon, silver or gold-based conductive materials. Carbon inks consist of graphite particles together with other ingredients that are responsible for dispersion and adhesion upon printing. The silver or gold inks are similar to these but contain a source of silver or gold instead of graphite. Some conductive inks also have a redox mediator on it, such as metals or metal complexes and pure organic polymers, as Meldola's Blue, Prussian Blue, cobalt phthalocyanine and nickel hexacyanoferrate, etc.

Such inks avoid using an external standard redox probe mediator to monitor the electrical output of the device. But the exact composition of all these commercial inks is crucial for a suitably printed material. It remains however a secret due to proprietary issues of the commercializing industries. Overall, the printed surface should be capable of following reproducible modification by different biomolecules, such as protein, antigens, antibodies, enzymes, membrane receptors or nucleic acids, acting as the biorecognition element in biosensors. Enzymatic biosensors are the most common, accounting the current needs for glucose monitoring in diabetes.

The substrate where the inks are printed is another relevant issue. In general, SPEs are typically designed on different supports, mostly plastic, as PET or PVC, and ceramics. These supports are expected to display insulating properties, because electrical flow should be driven only through the conductive inks printed on top.

In general, these materials work well on SPEs for POC applications, being the plastic-based ones less robust and cheaper than ceramic-based devices. However, an extended worldwide use of such SPEs with synthetic and non-biodegradable support materials pose environmental concerns. A truly worldwide application of POC devices requires replacing such materials by a naturally-derived compound, provided that this natural compound confers proper electrical and mechanical stability features to the final POC device.

In addition to this, the electrical features of the electrodes obtained by these methods are crucial for the analytical performance of the final biosensor. In general, the better the conductivity of the electrodes on the substrate, the better the analytical behaviour of the final device.

However, the electrode material should also be compatible with the substrate, and remain well adhered and stable for long time, with excellent conductivity features. This may also decrease the number of ink layers used to generate each electrode. Thus, an excellent substrate should be a strong isolating material and structurally compatible with the ink used to prepare the electrodes, besides being a naturally derived compound, promptly available.

Moreover, several works reported that different types of nanostructures can yield improvements in the analytical performance of biosensor, in terms of sensitivity or detection limit. The main explanation for this improvement is geometry/roughness, which may lead to an increased surface area, thereby enhancing the electrocatalytic activity of the working electrode.

EP1150118 describes a biosensor comprising an electrically insulating base plate, an electrode system that is mounted on the base plate and includes at least a working electrode and a counter electrode, wherein a silver paste is screen-printed on an electrical insulating base (or substrate) composed of polyethylene terephthalate (PET).

Although polyethylene terephthalate (PET) is inexpensive, the adhesion of the ink to this substrate demands specific conditions, due to its highly hydrophobic and flat nature. With little roughness and poor reactivity with other compounds, there is a low adhesive force of the printed ink on these substrates. This weak adhesion of the ink further leads to large waste of materials, increasing the costs of production.

Moreover, the time for zero-plastics is coming and this is an uncovered way of disposing plastic-based material into the environment. Finally, the use of a silver paste turns out the overall process much more expensive.

EP0969281 describes an electrode strip, for the direct analysis of electroactive species comprising a working electrode formed by a non-mercury-based metal ink screen-printed onto a plastic or ceramic support.

The use of a plastic and ceramic supports has the same problems as a PET support, mainly deriving from their poor adhesion to many ink materials and for posing serious environmental concerns after being disposed of. The cost of a ceramic-based device is also higher than the one in plastic.

EP1353169 describes a biosensor comprising: an electrically insulating base plate; an electrode system comprising a working electrode and a counter electrode formed on said base plate; a cover member which is joined to said base plate to form a sample solution supply pathway for supplying a sample solution to said electrode system between the cover member and the base plate; and a **reagent system** comprising at least an oxidoreductase and an electron mediator disposed in said sample solution supply pathway, wherein said electron mediator is provided on said base plate and said oxidoreductase is provided on said cover member such that said electron mediator and said oxidoreductase are not in contact with each other, and wherein at least one of the **electron mediator and the oxidoreductase** is carried by a **carrier** being said carrier selected from the group consisting of filter paper, glass filter, cellulose fibre, paper and **cork.**

However, in this biosensor the cork only acts as a carrier of the reagent system. This is due to the porous properties of the cork material to be able of carrying reagents inside or on its surface, thus not related to any insulating and rough features electrochemical properties of this type of material.

Moreover, the electrochemical cell described in this is limited to two-electrodes, being therefore applied only the specific electrical-based detection systems.

Therefore, there is a need to overcome the above-mentioned drawbacks of the prior art. For this purpose, the present invention provides a novel support with a two- or three-electrode system on a cork substrate, where the conductive ink is printed.

These supports may be further used to produce biosensors by modifying the working electrode area with the appropriate biorecognition element. Thus, such supports have wide interest in the production of biosensors for POC devices, providing the required electrical and mechanical stability features of such devices, whilst maintaining high reproducibility. Moreover, the production and the dispose of these devices are environmentally compatible and can be made in an easy and less expensive way than the conventional ones.

**Summary of the invention**

The present invention relates to a **cork substrate** for printing electrodes, to a **cork support** comprising electrodes for producing sensing surfaces intended for the electrical transduction of biomolecules, to a **biosensor** comprising a cork support and to a **process** for obtaining said substrates, supports and biosensors.

In a first aspect of the present invention, the cork substrate is a laminated cork material in which suitable conductive inks are printed according to claim 1.

The use of cork for the purposes of producing a substrate for biosensors favours the ink adhesion and may reduce the number of ink layers required to produce electrodes with given electrical conductivity. The rough morphology of cork seems to be the most relevant physical property favouring the adhesion of the ink to this substrate (when compared to other substrates), reducing the amount of wasted materials during production. Moreover, as the biosensors to be produced on these supports are typically disposed of after each measurement, the use of a cork-based substrate ensures an environmentally friendly approach.

In a second aspect, the present invention relates to a cork support comprising an electrode printed on a cork substrate for biosensors according to claim 3.

The electrodes printed on cork are designed with conductive inks by means of screen-printed technology. Two- or three-electrode systems may be established, combining working, auxiliary and/or reference electrodes.

The electrodes have increased electroactive areas due to the hydrophobic and rough features of the cork substrate. This is especially relevant when the working electrode is considered. In this case, this feature also enhances the electron transfer capabilities of the electrode at time of measurement. Since the electrochemical signal is related with the electrical properties of the electrodes, when the overall conductivity increases, a biosensor assembled on it shall become more sensitive to low concentrations.

In another aspect, the present invention relates to biosensors comprising screen-printed electrodes on a cork substrate according to claim 6.

Biosensors combining printed electrode systems are ideal for POC analysis, which is especially relevant in disease screening and environmental monitoring. These devices allow the use of reduced sample volumes, typically less than 50 $\mu$L. Biosensors with electrodes printed on cork supports offer the advantages of further decreasing costs, in terms of support and/or ink consumption/requirements, while improving the sensitivity and detectability features (for ensuring better electrical output signals). Moreover, these biosensors may be used and disposed of without special environmental concerns, when compared to the current commercial devices.

In another aspect, the present invention relates to a process for obtaining a cork substrate according to claim 8.

The substrate used herein comprises a laminated form of cork. This cork may be either obtained from the oak tree or from cork wastes. This is further covered by a thin-film of a hydrophobic material, to ensure hydrophobicity and homogeneity among the different laminated cork supports. This substrate is a low-cost material, also allowing recycling wastes from the cork industry, while ensuring the production of environmentally friendly supports for biosensors.

In another aspect, the present invention relates to a process of obtaining cork support comprising printed electrodes on a cork substrate according to claim 11.

The electrodes can be hand-printed on cork by using a mask that defines the number of electrodes, their area, sizes and overall disposition. The mask is placed on top of the cork substrate and the ink is casted there by moving a glass roll through this mask. Several layers of ink may be printed to ensure suitable electrical features to the final electrodes.

A single plate of the mask may combine up to 75 electrodes. In general, the process of obtaining electrodes on cork substrates does not need a specialized equipment, like typical screen-printers. Moreover, this manufacture process becomes cheaper and easier than the other alternatives, since the substrate cork offers a good adhesion to the ink.

In a further aspect, the present invention relates to a process of obtaining biosensors having cork electrodes according to claim 14.

The cork supports modified by screen-printing inks may be further sensitized to a given biomolecule to produce a given biosensor. For this purpose, a biorecognition element may be tailored/deposited on the working electrode area.

Such biosensor offers the advantages of having better electrical output features than other systems prepared in a similar way but on other supports (e.g., ceramic or PET), which is expected to lead to higher sensitivity and lower detection limits.

**Description of the figures**

**Figure 1**

**A)** Schematic representation of a conventional SPE, and
**B)** the photograph of a SPE using cork as support material, according to an embodiment of the invention.

**Figure 2**
Mask drawing for a single for three-electrode systems using electrodes with the same ink (A) and electrodes with a different ink (B).

**Figure 3**
Cyclic voltammograms of cork, PET and ceramic supports, from -0.3V to +0.7V, at 50.0 mV/s scan rate, in solutions of $Fe(CN)^{3-/4-}$ prepared in 100.0 mM KCl, with different concentrations (from 1.0 to 10.0 mM), wherein:

**A)** Cork [INVENTIVE]];
**B)** PET [COMPARATIVE];
**C)** Ceramic [COMPARATIVE].

**Figure 4**
Cyclic voltammograms of PEDOT modified cork supports prepared according to the embodiment, from -0.3V to +0.7V, in solution of $[Fe(CN)_6]^{3-/4-}$ (A) or $[Ru(NH_3)_6]^{2+/3+}$ (B) with a 5.0 mM concentration and prepared in 100.0 mM KCl, at different scan rates (from 25 to 500 mV/s).

**Figure 5** - Voltammograms of sulfadiazine standard solutions from 8 to 152 pg/mL, using the (A) cork substrate, and (B) the corresponding calibration curves, on the (a) cork substrate or (b) on the carbon commercial SPE from Dropsens.

**Figure 6** - Nyquist plots (left) of the CEA biosensor in a 5.0 mM standard redox probe of $[Fe(CN)_6]^{3-/4-}$, prepared in phosphate buffer saline, after incubating the WE in increasing concentrations of CEA standard solutions (0.0001 to 0.5 ng/mL), and (right) the corresponding calibration curve.

**Description of the invention**

The present invention relates to a cork substrate for printing electrodes, to a cork support comprising printed electrodes for producing sensing surfaces intended for the electrical transduction of biomolecules, to a biosensor comprising a cork substrate and to a process for obtaining said substrates, supports and biosensors.

Cork is a natural compound that may act as a good support to produce SPEs for POC applications due to several reasons. It is naturally hydrophobic, has low cost, is readily available and displays good electrical and thermal insulation features, while providing high mechanical robustness. It also allows controlled chemical modification. Cork is composed mainly by polymeric structures and extractives with hydroxyl, aldehyde/ketone and/or carboxylic acid functions. After oxidation, these groups may be activated by well-known carbodiimide chemistry, allowing mild modification procedures. Overall, a suitably conductive material may be assembled or casted on top of a planar cork support, making use of physical or chemical deposition methods.

Due to these properties of cork, studies were performed to evaluate and assess the possibility of using cork substrates to obtain electrodes useful to produce biosensors.

In the scope of the present invention the expression "substrate" applies to laminated or hydrophobic cork materials.

In the scope of the present invention the expression "support" applies to cork material with printed electrodes, thus "support" comprises a "cork substrate" and a printed electrode.

Cork substrates proved to be a viable material for the integration of electrically conductive layers using the screen-printing technique for (bio)sensing purposes.

The laminated cork is from the natural material collected from an oak tree or a waste material from the cork industry that is compressed and laminated for different purposes.

**1. Preparation of cork substrates**

Cork material can be obtained from cork industry or from commercially available suppliers. When the support is directly produced from the cork collected from the tree, the cork is directly laminated in plates of specific sizes and thicknesses. Alternatively, the cork collected from the trees by the cork industries is mostly used to produce bottle stoppers, which generates substantial cork wastes. The support may be produced from these wastes, by granulating these into different sizes, and agglomerated these after into different plates, with different thicknesses and sizes, depending on their specific application. The material used to agglomerate the cork granules depends on the formulation used by the industry producing these. Several ways are known from the art to produce cork laminates suitable to use in the scope of the present invention such as the one disclosed in: https://www.youtube.com/watch?v=R-YWAcmGj_s

Cork laminates used herein have a variable thickness of 0.08 to 0.12 cm, providing either from the oak tree material or from the industry wastes. The industry wastes correspond to materials generated along the production of different cork-based products, which are agglomerated after in a laminar plate.

When these laminates are not water proof, for example after incubating the complete electrode by 30 minutes in water, the cork laminates are further ensured hydrophobic by applying a coating with a waterproof film. Suitable materials for this film may be selected from polysiloxane, fluoro alkylsilane, fluoropolymer, resin-based polymer, or naturally derived polymer, such as cellulose modified by acylation or bee wax. In a preferred embodiment the film is an epoxy-resin solution.

This film can be casted on the laminated cork for example by spin-coating, dip-coating or doctor blading methods. After drying, the hydrophobic cork-supports are ready to print electrodes.

## 2. Preparation of cork supports with printed electrodes

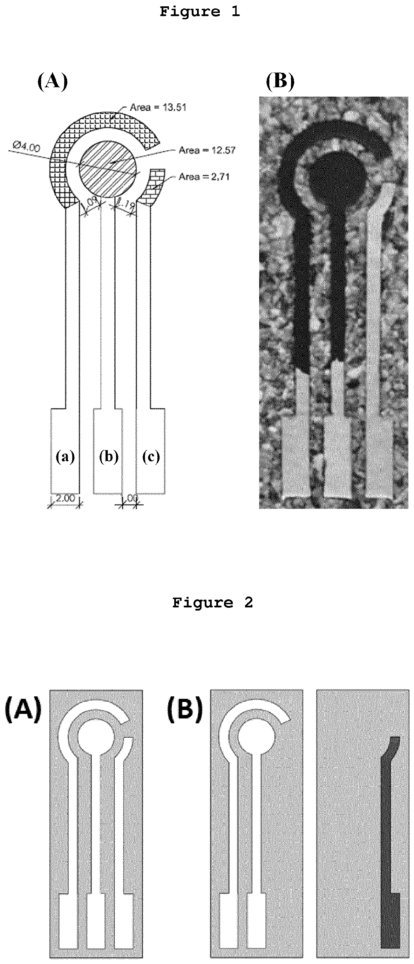

The electrodes can be designed by homemade screen-printing, using a mask with the negative image of the intended electrodes. In a simple view, this mask contains the negative drawings of the desired electrodes. Several masks can be employed for this purpose, having as target the two- or three-electrodes systems, as shown in Figure 1a.

As an example, two possible masks are shown in Figure 2, using electrodes with the same ink, in which a single mask is employed, figure 2A, or having two different inks applied, e.g. for a reference electrode with a different material, as shown is Figure 2B.

Suitable inks for producing electrodes are commercially available inks such as carbon or silver inks. Several electrodes can be produced in two- or three-electrode conventional systems, or other number arrangements, with different electrode sizes and dimensions, to be employed in function of the intended purpose.

In a preferred embodiment, this includes having two- or three-electrode systems, from which the working electrode (WE) area exposed to the sample can vary in the range of 1 mm$^2$ to 30 mm$^2$; the area of the auxiliary electrode (AE) is at least the same as that of the WE; and the area of the reference electrode (RE) is set a minimum value, varying for example in the range of 1 mm$^2$ to 4 mm$^2$.

The electrodes can be assembled in multiple units in a single plate that may allow printing up to 75 electrodes. The electrode system may also include more than one working electrode.

Once the mask is placed on top of the hydrophobic cork, the ink is applied on this surface and spread with for example a spatula/glass roll. The number of layers of ink used for this purpose is related with the electrical features intended for each electrode and for the overall device, which typically varies from 1 to 6. In practice, a regular multimeter should signal the passage of current through each individual electrode present on the hydrophobic cork substrate.

Each ink layer is then cured, for example in an oven, typically at 25-60°C, for 20-60 min. After, the mask is removed.

To further improve the electrical features of the resulting biosensor, the working electrode area may be modified with a conductive polymer support, which may be prepared in-situ by electropolymerization. All conductive polymers prepared by electropolymerization may be employed for this purpose. Poly (3,4-ethylenedioxythiophene), PEDOT, is highlighted herein, by testing it in the examples shown after.

## 3. Preparation of a biosensor comprising an electrode based on cork substrate

Biosensors may be applied in a wide range of different areas. Typically, a biosensor combines a (bio)chemical component (such as protein, antigens, antibodies, enzymes, membrane receptors, nucleic acids, etc.), ensuring the recognition of a target biomolecule. This component is placed on the working electrode and the subsequent recognition is transduced herein by electrochemical readings of the cork substrate containing the three-electrode systems.

The (bio)chemical component may be immobilizing on working electrode surface or produced on it. One of the possibilities herein is to tailor a molecularly-imprinted polymer for a given biomolecule. In this, the monomers and the target molecule are placed on the three-electrode system and a suitable potential approach is applied into these to allow the production of a suitable polymeric layer. The target molecule is extracted after to generate vacant sites with complementary shape and to which the target molecules in a sample may rebind after.

Suitable monomers can be selected from pyrrole, 3,4-ethylenedioxythiophene, thiophene, aniline, benzoic acid, gallic acid, phenylenediamine, aminophenol, dopamine, eriochrome black T, methylene blue, or any of their derivatives containing one or more different chemical functions among carboxylic acid, ether, amine, hydroxyl, thiol, phosphate, and sulphonate, or aromatic groups, in different positions.

Antibiotics in water are today an issue of great concern. Humans have spread antibiotics throughout the environment and are contributing to the generation and a new era of antibioticresistant species that may decimate the overall population in the next decades. Thus, it is very important to reduce the levels of antibiotic contamination in water, deriving mainly from wastewater treatment plants and from fish culture activities, for which a suitable device for routine monitoring is highly appreciated.

Therefore, in a preferred embodiment of the invention a biosensor for screening antibiotics is developed by using a cork support of the invention. The biorecognition element used herein can be assembled by molecularly-imprinted technology for example by electropolymerizing. In the case of a biosensor for screening antibiotics it is produced an imprinted polymer, for example of a polypyrrol polymer generated on a PEDOT layer in the presence of a given antibiotic. The electrochemical performance of the resulting biosensor can be evaluated by incubating it in standard solutions of the target element and after monitoring the behaviour against a standard redox probe.

Cancer biomarkers are biomolecules that circulate in body fluids and that signal the installation or progression of the cancer diseases. Their relevance outcomes from their easy access, due to their circulation in body fluids, allowing routine screening by non-invasive procedures. One of the cancer biomarkers in use in clinical practice is CEA, which is a glycoprotein involved in cell adhesion that has altered values in several cancer conditions.

Thus, in another preferred embodiment a biosensor for screening a cancer biomarker is developed by using a cork support of the invention. The biorecognition element used herein can also be assembled by molecularly-imprinted technology, such as by electropolymerizing eriochrome black-T (EBT) in situ and on top of a conductive layer such as a PEDOT layer. The electrochemical performance of the resulting biosensor was evaluated by incubating it in standard solutions of the target element and after monitoring the behaviour against a standard redox probe.

**4. Current and voltage measurements of devices**

The electrical features of the cork substrate with the conductive printed two- or three-electrode system, or other arrangement, may be assessed by following the behaviour of a standard electrochemical reaction. In general, the anodic and cathodic peak currents, the peak potentials, and the scan-rate of the applied potential are followed for this purpose. Impedance data may also be used. These are followed to establish valuable information with regard to the electrical features of the resulting cork-based electrodes and make the following considerations.

4.1 Electroactive electrode area

To evaluate the electroactive area of the WE, a potential can be applied to the working electrode continuously varying within time (scan-rate) to produce the oxidation/reduction reactions of the electroactive species present. In case of $[Fe(CN)_6]^{3-/4-}$, the potential was scanned from -300 mV to 700 mV, and in $[Ru(NH_3)_6]^{2+/3+}$, the potential was scanned from -500 mV to 100 mV. The effect of scan-rate ($v$) on the peak current ($Ip$) was obtained by Randles-Sevcik equation,

$$I_p = (2.69 \times 10^5)\, n^{3/2}\, ACD^{1/2}\, v^{1/2} \qquad \textbf{(eq.1)}$$

where $n$ is the number of electrons involved in the reaction, $A$ is the electroactive area of the electrode (cm$^2$), $D$ is the diffusion coefficient of the redox specie (cm$^2$.s$^{-1}$), $C$ its bulk concentration (mol.dm$^{-3}$), $v$ is the potential scan-rate (V.s$^{-1}$), and Ip is the peak current (A).

When the electrochemical systems under testing involves the diffusion of the electroactive species on the surface, there is a linear dependency between $I_p$ and $(V)^{1/2}$. Moreover, this equation allows to calculate electroactive area ($A$) of each electrode, which it demonstrated in Table 3,4 and 5.

Overall, CORK substrate had a higher active area (9.00 mm$^2$) compared with CER-WP (7.00 mm$^2$) and PET-WP (8.00 mm$^2$).

4.2 Separation of the redox peaks

The standard redox probe tested herein involves a reversible reaction, for which the potential difference of the redox peaks ($\Delta E_p$) should be equal to 59/n mV, where n is the number of electrons transferred in the redox event. However, in SPEs these reactions do not behave as a truly reversible system, and an increment of $\Delta E$ with scan rate is normally observed. This increment is greater for less reversible systems, meaning that the conductive systems in the SPEs are worse.

One possibility to improve this behaviour is SPEs consists in the modification of the WE with a conductive film. Poly(3,4-ethylenedioxythiophene), PEDOT, is a typical polymer used for this purpose and is amongst the most stable conducting polymers currently available. It is of easy and low cost deposition, offering good thermal and electrochemical stability and high conductivity.

Overall, cork substrates CORK proved to be a viable substrate for the integration of an electrically conductive layer using the screen-printing technique. In alternative of this technique a deposition onto cork substrates was made more simply and cheaply.

This may be explained due to the high porosity presented by cork comprised in the inventive substrate CORK that allows it to exhibit a low mass density and high surface area, obtaining a large surface area available for printed ink. Thus, the ink presents a carbon layer with some voids throughout the surface, where it may be enhancing electrical behaviour through the surface area available for reaction.

In summary, a new electrode support for electrochemical was fabricated based on screen-printed electrode and compared with others available commercial support by CV measurements and two distinct redox probes. Experimental results obtained in the present study demonstrated that cork support exhibited better electrochemical response and sensitivity, in comparison with PET and Ceramic. Besides that, it was observed that electrochemical performance was improved after introduction PEDOT onto electrode surface.

In overall, cork supports show a good reproducibility results in all electrochemical measurements and this study show that these electrodes are promising for practical application of a biosensor based on SPE.

## EXAMPLES

### Example 1. Preparation of cork substrates

Cork laminates having a variable thickness of 0.08 to 0.12 cm were obtained from a cork supplier. These laminates presented pores through which the water could diffuse and allow an electrical contact between the electrical paths and electrodes that are to be printed on the substrate. This was confirmed by incubating the complete electrode by 30 minutes in water.

To avoid this, a water-proof (WP) layer was casted on it. The WP layer was produced by spin coating a solution of a hydrophobic liquid, diluted in 10-70%, for 60-180s, at 100-2000 rpm. After this, the substrate was dried at room temperature, overnight.

### Example 2. Preparation of cork supports

Cork supports (CORK) were produced based on cork substrates prepared as described in Example 1, modified or not with a WP film to ensure hydrophobicity, and printed with conductive inks, making use of a mask. Figure 1 shows a representation of a cork support, wherein the electrodes printed on top combined a three-electrode system, with a working electrode (WE), an auxiliary electrode (AE), and reference electrode (RE) as represented in Figure 1.

The areas of WE, AE and RE used in this specific design were 12.57, 13.51 and 2.71 mm$^2$, respectively.

Two kind of inks were employed herein to design the different electrodes and their conductive paths: a silver ink was used for printing the RE, and a carbon ink for designing the WE, AE and RE, as shown in Figure 1. The carbon ink is spread over a mask containing the negative image of electrodes to be printed (Figure 2). The inks were used in a conventional way as known by the skilled person.

In the three-carbon electrode system, the carbon ink was spread in the mask in Figure 2A. For obtaining a silver RE, the inks were casted separately in two stages.

The mask used is represented in Figure 2B, in which the carbon ink was spread on the left image (WE and AE) and the silver ink was spread in the right image (RE). Alternatively, the carbon ink can also be spread in the mask of figure 2A and the silver ink in the RE, placed on top of the carbon ink with the help of mask in Figure 2B-right.

The electrical contacts were also covered with silver ink to ensure good electrical contact with the electrical reading box.

### Example 3. Preparation of conventional supports

For comparison purposes, conventional supports based on polyethylene terephthalate (PET) and ceramic (CER) substrates, with WP film (PET-WP) and (CER-WP) or without a WP film (PET) were prepared in the same way as the cork supports based (COR) on cork substrates with (COR-WP) or without a WP film.

### Example 4. Electrochemical features of the cork supports

In order to assess the electrical performance of the cork substrates of the invention [INV] cork supports (CORK) prepared according to Example 2 were used to prepare electrochemical biosensors for any given target biomolecule, and their electrochemical performance was evaluated against the standard redox probe $[Fe(CN)_6]^{3-/4-}$.
These studies were conducted by cyclic voltammetry, using different concentrations of the probe.

The electrochemical performance was evaluated by recording cyclic voltammograms at different concentrations of a standard redox probe $[Fe(CN)_6]^{3-/4-}$, prepared in varying concentrations (1.0, 2.5, 5.0, 7.5 and 10.0 mM), in an electrolyte solution of 100 mM of KCl, for a scan rate of 50 mV/s.

### Example 5. Comparison of electrochemical features of cork supports [INV] vs. conventional supports [COMP]

In order to compare the electrical performance of the cork substrates (CORK) of the invention [INV] and the conventional supports [COMP] prepared respectively according to Example 2 and Example 3, said supports were used to prepare electrochemical biosensors for any given target biomolecule being their electrochemical performance evaluated against the standard redox probe $[Fe(CN)_6]^{3-/4-}$.

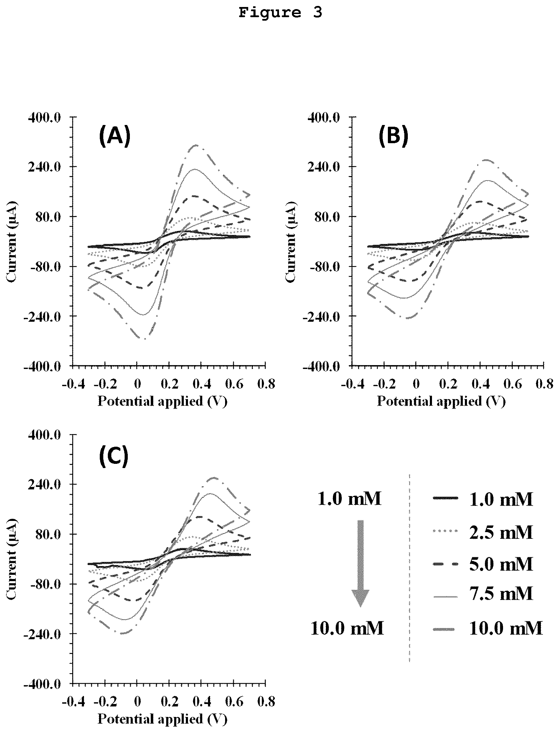

These studies were conducted by cyclic voltammetry, using different concentrations of the probe and the electrochemical performance of all supports was evaluated as described in Example 4. The results so obtained are shown in Figure 3A, wherein Fig.3a relates to a cork support (CORK) [INV], Fig. 3b to a PET support [COMP] and fig. 3c to a ceramic support [COMP].

The data corresponding to the studies having a 5.0 mM concentration of redox probe is listed in table 1. In general, the ratio of cathodic and anodic peak currents was 1.0, with a difference of potential peaks ($\Delta E$p) of 330 mV, and an active electrochemical area of 9.0 mm$^2$.

**Table 1 -** Electrochemical data extracted from the cyclic voltammograms of cork-, PET- and CER-based supports containing or not WP film, in 5.0 mM Redox Probe [Fe(CN)$_6$]$^{3-/4-}$, prepared in 100 mM KCl.

| Electrode type | Physical (WE, mm$^2$) | $I_{pa}$ (µA) | $I_{pc}$ (µA) | $I_{pa}/I_{pc}$ | Active area (WE, mm$^2$) | $E_{pa}$ (mV) | $E_{pc}$ (mV) | $\Delta E_p$ (mV) |
|---|---|---|---|---|---|---|---|---|
| CER | 12.6 | 142 | 137 | 1.0 | 6.0 | 448 | -94 | 542 |
| CER-WP | 12.6 | 135 | 134 | 1.0 | 7.0 | 392 | -23 | 415 |
| PET | 12.6 | 102 | 102 | 1.0 | 7.0 | 465 | -107 | 572 |
| PET-WP | 12.6 | 128 | 128 | 1.0 | 8.0 | 401 | -43 | 444 |
| Cork-WP | 12.6 | 145 | 150 | 1.0 | 9.0 | 360 | 30 | 330 |

These features confirmed the better electrochemical performance of the CORK supports [INV], when compared to equivalent conventional supports [COMP] prepared with PET and CER substrates (Figure 3B and 3C). The WP film and the inks were used in these were the same use in the CORK-WP, to enable a valid comparison. In general, the peak current values produced by the cork supports are higher and the differences in the peak potentials are smaller.

Moreover, it was also clear that the best behaviour of the cork support was not related to the presence of a WP film alone, as shown by a direct comparison of electrodes prepared with and without it (table 1).

Overall, all the data obtained comparing to concurring supports confirmed the electrochemical benefits of using cork as substrate.

## Example 6. Electrochemical features of the cork supports modified with a conductive polymer

The usefulness of the cork supports in the preparation of biosensors requires their smooth and effective modification. For this purpose, a first modification on this support comprised the addition of a conductive film of Poly(3,4-ethylenedioxythiophene), also known as PEDOT, to the WE of the same three-electrode system prepared as described in Example 2. This was made by electropolymerizing 3,4-ethylenedioxythiophene (EDOT) in-situ.

The electrochemical performance of the resulting PEDOT/cork support was evaluated against the standard redox probe [Fe(CN)$_6$]$^{3-/4-}$. These studies were conducted by cyclic voltammetry, using different concentrations of each probe and different scan-rates.

### 6.1 - Physical details of the PEDOT/cork support

The cork support was prepared as described in Example 1. After this, the three-electrode system therein was cleaned by covering these with a diluted sulphuric acid solution and applying 10 successive cycles from -200 mV to 1500 mV. Next, the PEDOT film was generated on top of the WE by electropolymerizing a 10.0 mM EDOT solution prepared in an electrolyte of 100.0 mM KCl. Electropolymerization was made by applying a potential of 1.10 V, for 40 s.

### 6.2 - Electrochemical performance of the PEDOT/cork supports

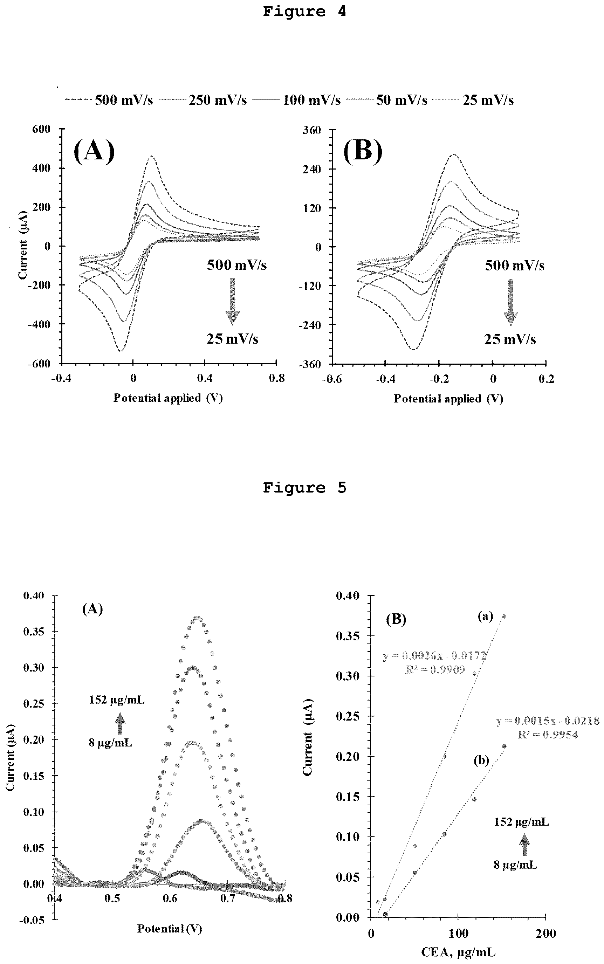

The electrochemical performance was evaluated by recording cyclic voltammograms of a 50.0 mM standard redox probe of [Fe(CN)$_6$]$^{3-/4-}$ or [Ru(NH$_3$)$_6$]$^{2+/3+}$, prepared in an electrolyte solution of 100 mM of KCl, and using varying scan-rates of 500, 250, 100, 50 and 25 mV/s. The results so obtained are shown in Figure 4.

The results related to [Fe(CN)$_6$]$^{3-/4-}$ are shown in Figure 4A. The average data obtained for the several scan-rates is listed in table 4. Compared to electrodes without this PEDOT layer, the ratio of cathodic and anodic peak currents decreased when PEDOT was present, but the peak currents and in the active area of the WE showed an exceptional increase. The difference between the peak potentials of the oxidation and reduction peaks was also substantially lower, from 421 mV to 100 mV. Moreover, the $E_p$ values for the Carbon/PEDOT WEs showed lower variability against the change in the scan-rate.

Overall, the results obtained with the [Ru (NH$_3$)$_6$]$^{2+/3+}$ probe are shown in Figure 4B, and were consistent with those obtained for the [Fe(CN)$_6$]$^{3-/4-}$ probe.

**Table 4 -** Average electrochemical data extracted from the cyclic voltammograms at varying scan-rates of Cork-WP supports with a WE of carbon or carbon with PEDOT, in 5.0 mM Redox Probe [Fe(CN)$_6$]$^{3-/4-}$, prepared in 100 mM KCl.

| WE | I$_{pa}$ (μA) | I$_{pc}$ (μA) | I$_{pa}$/I$_{pc}$ | Active area (WE, mm$^2$) | E$_{pa}$ (mV) | E$_{pc}$ (mV) | ∆E$_p$ (mV) |
|---|---|---|---|---|---|---|---|
| Carbon | 93.5±2.2 | 93.3±0.8 | 1.0±0.0 | 6.1±0.6 | 332.2±21.4 | 241.7±41.7 | 421.3±58.2 |
| Carbon/ PEDOT | 160.8±4.0 | 181.8±3.8 | 0.9±0.0 | 16.8±0.4 | 68.8±6.3 | -32.1±6.1 | 100.9±4.7 |

Overall, these features confirmed the possibility of modifying the WE of the cork-support in an easy way. The approach taken was also responsible for significant beneficial features in terms of electrochemical performance.

**Example 7. Biosensor for screening an antibiotic**

A biosensor for screening antibiotics was prepared by using a cork support of the invention. The biorecognition element used herein is assembled by molecularly-imprinted technology by co-electropolymerizing EDOT and Pyrrol in situ, and on top of the PEDOT layer. The electrochemical performance of the resulting biosensor was evaluated by incubating it in standard solutions of the target element and after monitoring the behaviour against a standard redox probe.

**7.1 - Physical details of the cork support**

The cork support was prepared as described in Example 2. After this, an imprinted polymer for sulfadiazine was produced on the PEDOT film by electropolymerizing Pyrrol (2.7mM) in the presence of sulfadiazine (5 mM), all prepared in 40 mM Britton-Robison buffer, pH 10. Electropolymerization was achieved by applying 20 successive cycles from -0.3 to 0.9 V, with a scan-rate of 50 mV/s. Sulfadiazine was removed by incubating the WE in a solution of 20 mM NaOH.

**7.2 - Electrochemical performance of the biosensor**

The electrochemical response of the biosensor to the presence of sulfadiazine was tested by incubating sulfadizine standard solutions of increasing concentrations, prepared in 40 mM Britton-Robison buffer, pH 10, in the WE area. For each concentration, the signal under differential pulse voltammetry (DPV) was collected. The sulfadiazine solutions ranged 8.0 to 152 pg/mL and were all prepared in 40 mM Britton-Robison buffer, pH 10.
The results obtained are shown in Figure 5. In general, it was possible to confirm that the current signals increased (Figure xA) for increasing concentrations of sulfadizine, revealing the electroactive behaviour of this compound and the ability of the MIP film to recognize it. This current increase was also displayed a linear behaviour against the sulfadizine concentration, was shown in Figure 5B.
For comparison purposes, the same bioreconition element was assembled on commercial carbon SPEs (from DropSens) prepared with the same PEDOT layer, and tested in parallel. In general, the highest sensitivity of biosensors prepared on the cork substrates was evident (Figure 5B), with calibrations displaying higher sensitivity (in 73%).

**Example 8. Biosensor for screening a cancer biomarker.**

A biosensor for screening a cancer biomarker was prepared by using a cork support of the invention. The biorecognition element used herein is assembled by molecularly-imprinted technology by electropolymerizing eriochrome black-T (EBT) in situ and on top of the PEDOT layer. The electrochemical performance of the resulting biosensor was evaluated by incubating it in standard solutions of the target element and after monitoring the behaviour against a standard redox probe.

**8.1 - Physical details of the cork support**

The cork support was prepared as described in example 2. After this, an imprinted poly(EBT) polymer was produced on the PEDOT film by electropolymerizing EBT (0.01 mM) in the presence of carcinoembryonic antigen (CEA, 200 µg/mL), all prepared in phosphate buffer saline, by applying 4 successive cycles from 0.0 to 0.95 V, with a scan-rate 50 mV/s. The rebinding cavities for CEA were generated after removing the CEA from the polymeric network. This was done by incubating overnight the WE surface in a 500 pg/mL solution of Proteinase K, and after in 100 mM solution oxalic acid for 1h.

**8.2** - **Electrochemical performance of the biosensor**

The electrochemical response of the biosensor to the presence of CEA was tested by incubating CEA standard solutions of increasing concentrations in the WE area and measuring the behavior of the three-electrode system after. This was done for a 5.0 mM standard redox probe of $[Fe(CN)_6]^{3-/4-}$, prepared in phosphate buffer saline, using electrochemical impedance spectroscopy (EIS). The CEA standard solutions ranged 1.0 to 500 pg/mL and were all prepared in phosphate buffer saline.

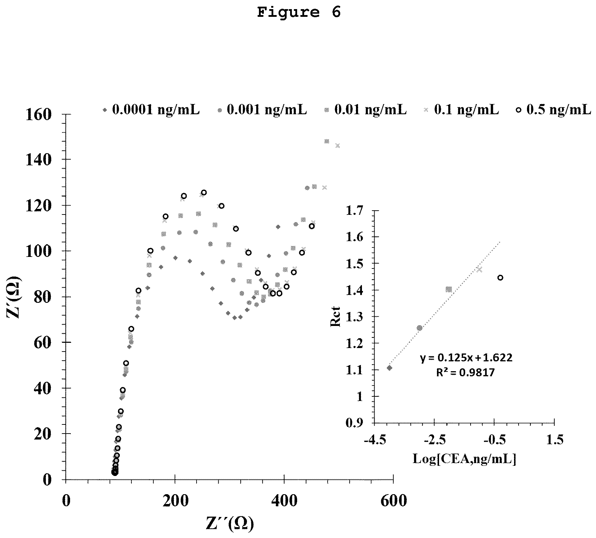

The results obtained are shown in Figure 6. In general, the data in the Nyquist plots revealed that the presence of CEA in higher concentrations yielded an increase in the resistance of the analysed surface due to the higher diameter of the semicircles observed. CEA is an isolating material and the increasing number of CEA molecules bound to the MIP surface increased the resistance of the surface to the charge-transfer of the redox probe. This behaviour was concentration dependent, having linear plots of resistance of charge-transfer (Rct) against the logarithm concentration of CEA (figure 6, right), thereby allowing the application of the biosensor in the determination of CEA.

**Claims**

1. A **cork substrate** for printing electrodes comprising a laminated cork material having a variable thickness of 0.08 to 0.12 cm, coated by a film of a hydrophobic-based material.

2. A cork substrate according to claim 1 wherein the hydrophobic-based material is selected from polysiloxanes, fluoro alkylsilanes, fluoropolymers, resin-based polymers, or naturally derived polymers.

3. A **cork support** for a biosensor comprising a cork substrate as described in any of the claims 1 or 2 and a printed electrode, wherein said electrode is printed with a conductive ink.

5. A cork support according to any of the claims 3 or 4 comprising a working electrode area modified with a conductive polymer support.

6. A **biosensor** for sensing surfaces intended for the electrical transduction of biomolecules comprising a cork support as described in any of the claims 3 to 5 and a working electrode having a biorecognition element.

7. A biosensor according to claim 6 wherein the biorecognition element is a molecularly imprinted polymer having as target an antibiotic marker or a cancer marker.

8. A **process for producing a cork substrate** for printing electrodes comprising the following steps:

   a) Providing a cork material in powder, granulate or compressed form;
   b) Laminating the cork material to obtain a laminate having a 0.08 to 0.12 cm thickness;
   c) Coating the cork laminate of (b) with a hydrophobic-based solution of a hydrophobic coating;
   d) Drying the coated cork laminate of (c).

9. A process, according to claim 8 wherein the cork material is laminated (a) directly from the material obtained from the oak tree and/or (b) from the waste products produced by the oak industry.

10. A process, according to any of the claims 8 to 9, wherein the cork laminate is coated with a hydrophobic-based solution of a hydrophobic coating selected from polysiloxanes, fluoro alkylsilanes, fluoropolymers, resin-based polymers, or naturally derived polymers, preferably with an epoxy-resin polymer by a spin coating, dip-coating or doctor blading technique.

11. A **process for producing a cork support for** a biosensor comprising the following steps:

   a) Providing a cork substrate, as described in any of the claims 1 or 2;

b) Printing an electrode on a cork substrate of (a) by a screen-printing technique applying a conductive ink to one or more masks with the negative image of the intended electrode placed on the top of the cork substrate of (a).

**12.** A process, according to claim 11, wherein two or three masks are used in the screen-printing of step (b) and wherein the same ink is applied to the masks and cork substrate in the screen-printing of step (b) or wherein a different ink is applied to the masks and cork substrate in the screen-printing of step (b), and said ink is applied in more than one layer of ink or inks to one or more masks and cork substrate.

**13.** A process, according to any of the claims 11 or 12, further comprising the modification of the working electrode area with a conductive polymer support.

**14.** A **process for producing a biosensor** comprising the following steps:

a) Providing a cork support as described in any of the claims 3 to 5; and
b) Immobilizing a biorecognition element on the working electrode surface or produce a biorecognition element on the working electrode surface.

**15.** A process according to claim 14 wherein the biorecognition element is a molecularly imprinted polymer, prepared in-situ by electropolymerization of suitable monomers being said monomers preferably selected from pyrrole, 3,4-ethylenedioxythiophene, thiophene, aniline, benzoic acid, gallic acid, phenylenediamine, aminophenol, dopamine, eriochrome black T, methylene blue, or any of their derivatives containing one or more different chemical functions among carboxylic acid, ether, amine, hydroxyl, thiol, phosphate, and sulphonate, and/or aromatic groups, in different positions.

Figure 1

Figure 2

Figure 3

Figure 4

Figure 5

Figure 6

**Europäisches Patentamt**
**European Patent Office**
**Office européen des brevets**

# EUROPEAN SEARCH REPORT

Application Number

EP 19 16 1929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/017939 A1 (AMORIM REVESTIMENTOS S A [PT]) 30 January 2014 (2014-01-30) * claim 1; figure 1 * | 1,2,8-10 | INV. B32B9/02 G01N27/327 G01N33/543 |
| Y | FERREIRA NÁDIA S ET AL: "New electrochemically-derived plastic antibody on a simple conductive paper support for protein detection: Application to BSA", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 243, 15 December 2016 (2016-12-15), pages 1127-1136, XP029915563, ISSN: 0925-4005, DOI: 10.1016/J.SNB.2016.12.074 * the whole document * | 1-15 | |
| Y | EP 0 109 852 A2 (UNIV CARDIFF [GB]) 30 May 1984 (1984-05-30) * page 3, line 21 - line 25; figure 1 * | 1-15 | |
| Y,D | EP 1 353 169 A1 (MATSUSHITA ELECTRIC IND CO LTD [JP]) 15 October 2003 (2003-10-15) * paragraph [0026]; claim 7 * | 1-15 | TECHNICAL FIELDS SEARCHED (IPC) B32B G01N |

The present search report has been drawn up for all claims

| Place of search | Date of completion of the search | Examiner |
|---|---|---|
| Munich | 13 May 2019 | Hinchliffe, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 16 1929

This annex lists the patent family members relating to the patent documents cited in the above-mentioned European search report.
The members are as contained in the European Patent Office EDP file on
The European Patent Office is in no way liable for these particulars which are merely given for the purpose of information.

13-05-2019

| Patent document cited in search report | | | Publication date | Patent family member(s) | | Publication date |
|---|---|---|---|---|---|---|
| WO 2014017939 | A1 | | 30-01-2014 | PT 106466 A<br>WO 2014017939 A1 | | 27-01-2014<br>30-01-2014 |
| EP 0109852 | A2 | | 30-05-1984 | NONE | | |
| EP 1353169 | A1 | | 15-10-2003 | CN 1486422 A<br>EP 1353169 A1<br>JP 3971997 B2<br>JP WO2002057767 A1<br>US 2004069628 A1<br>WO 02057767 A1 | | 31-03-2004<br>15-10-2003<br>05-09-2007<br>27-05-2004<br>15-04-2004<br>25-07-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1150118 A
- EP 0969281 A
- EP 1353169 A

**Non-patent literature cited in the description**

- **DR. GUILLAUME CHANSIN.** OLED Display Forecasts 2015-2025: The Rise of Plastic and Flexible Displays. *IDTechEx Research Report,* 2015